# EUROPÄISCHE PATENTSCHRIFT

(11) **EP 3 046 509 B1**
(45) Veröffentlichungstag und Bekanntmachung des Hinweises auf die Patenterteilung: **07.02.2018**
(21) Anmeldenummer: 14838859.8
(22) Anmeldetag: 15.12.2014
(51) Int. Cl.: A61F 2/14, A61F 2/00

(54) **VORRICHTUNG ZUR LAGERUNG UND ZUM TRANSPORT EINES TRANSPLANTATS ODER IMPLANTATS**
DEVICE FOR SUPPORTING AND TRANSPORTING A GRAFT OR IMPLANT
DISPOSITIF DE STOCKAGE ET DE TRANSPORT D'UN TRANSPLANT OU D'UN IMPLANT

(43) Veröffentlichungstag der Anmeldung: 27.07.2016
(73) Patentinhaber: Geuder AG, 69126 Heidelberg (DE)
(72) Erfinder: SZURMANN, Peter, 66123 Saarbrücken (DE); FATH, Hartmut, 69168 Wiesloch (DE)
(74) Vertreter: Patent- und Rechtsanwälte Ullrich & Naumann
(86) Internationale Anmeldenummer: PCT/DE2014/200717
(87) Internationale Veröffentlichungsnummer: WO 2016/095884

(56) Entgegenhaltungen:
- WO-A1-2009/050511
- WO-A2-2005/037136
- DE-U1-202011 106 789
- US-A1- 2003 214 139

## Beschreibung

Die vorliegende Erfindung betrifft eine Vorrichtung zur Lagerung und zum Transport eines Transplantats oder Implantats, mit einem mit einem Medium, vorzugsweise Nährmedium, befüllbaren Behälter, mit einer in dem Behälter anordenbaren Aufnahmeeinrichtung für das Transplantat oder Implantat, wobei die Aufnahmeeinrichtung einen Aufnahmeraum für das Transplantat oder Implantat und zwei zu dem Aufnahmeraum führende Durchgänge aufweist, von denen mindestens ein Durchgang für ein Ein- und/oder Ausbringen des Transplantats oder Implantats in den oder aus dem Aufnahmeraum dimensioniert ist, und mit jeweils einer Verschlusseinrichtung für die beiden Durchgänge, von denen mindestens eine Verschlusseinrichtung für das Medium durchlässig ist. Vorzugsweise sind zum Transport beide Verschlusseinrichtungen für das Transplantat oder Implantat undurchlässig.

Des Weiteren betrifft die vorliegende Erfindung eine entsprechende Aufnahmeeinrichtung sowie ein Set mit einer wie oben beschriebenen Vorrichtung in sterilisiertem Zustand oder in einer zur Sterilisierung geeigneten Verpackung.

Die Transplantation der menschlichen Hornhaut gehört zu den am häufigsten und erfolgreichsten durchgeführten transplantationschirurgischen Eingriffen. Die Transplantation der Hornhaut unter Verwendung aller ihrer Schichten (Epithel, Bowman-Membran, Stroma, Descemet-Membran und Endothelzellschicht) im Rahmen einer perforierenden Keratoplastik ist seit über 100 Jahren bekannt. Mit dieser Operationsmethode werden im Allgemeinen gute Ergebnisse erzielt, jedoch ist die Rekonvaleszenz sehr langwierig und eine Visuserholung kann üblicherweise endgültig erst nach Entfernung des zweiten Fadens erreicht werden. Dies kann eine Zeitspanne von bis zu 18 Monaten nach dem Eingriff ausmachen.

Gut die Hälfte aller Hornhauttransplantationen wird wegen Erkrankungen des Hornhautendothels durchgeführt. Für diese Erkrankungen würde sich prinzipiell ein schichtspezifischer Ersatz der Hornhaut eignen. Geeignete Techniken hierfür liegen mittlerweile vor. Durch die Transplantation der Descemet'schen Membran mit einer anhängenden Stromalamelle (DSAEK) kann seit einigen Jahren eine im Vergleich mit der perforierenden Keratoplastik viel schnellere Visuserholung und demzufolge eine bessere Patientenzufriedenheit erreicht werden.

Zur Therapie bei Erkrankungen der Hornhaut, welche das Hornhautendothel betreffen, ist des Weiteren eine neuartige, spezielle Form der Hornhauttransplantation beschrieben worden. Dabei handelt es sich um die Descemet Membrane Endothelial Keratoplasty (DMEK). Im Zuge eines solchen Eingriffs können die erkrankten Endothel-Zellen einschließlich der darunter liegenden Descemet-Membran entfernt und durch eine Descemet-Membran mit gesundem Hornhautendothel eines Spenders ersetzt werden. Auf eine perforierende Keratoplastik kann dabei verzichtet werden, vielmehr lässt sich das Transplantat durch eine vergleichsweise kleine Inzision in die vordere Augenkammer transferieren und durch sehr vorsichtige Manipulation ausbreiten. Danach wird das Membran-Präparat mittels Luftzugabe am hinteren Stroma fixiert.

Eine erfolgreiche DMEK-Operation ermöglicht dem Patienten eine sehr schnelle visuelle Rehabilitation. Es ist davon auszugehen, dass der Patient schon nach ca. zwei Monaten nach dem Eingriff eine volle Sehschärfe erreicht.

Allgemein sind eine ganze Reihe chirurgischer und insbesondere ophthalmologischer Eingriffe bekannt oder denkbar, bei denen ein Transplantat oder ein Implantat bereitgestellt und in den lebenden Körper eingebracht wird. Dabei ist es im Sinne einer raschen Rekonvaleszenz des Patienten - wie oben anhand der DMEK-Operation beispielhaft ausgeführt - vorteilhaft, ein Transplantat oder Implantat durch eine Inzision begrenzter, und insbesondere möglichst kleiner Größe in den lebenden Körper einzubringen.

Aus der DE 10 2010 051 458 A1 ist beispielsweise eine Vorrichtung zum Bereitstellen und zum Einbringen eines Transplantats oder eines Implantats in den lebenden Körper, insbesondere für ophthalmologische Eingriffe, bekannt. Die hier beschriebene Vorrichtung weist eine Kartusche oder Hülse mit zwei gegenüberliegenden, endseitigen Öffnungen auf, wobei diese Kartusche oder Hülse in besonderer Weise geeignet ist, ein Transplantat oder Implantat durch eine sehr kleine Inzision in den Körper eines Patienten einzubringen, wie dies beispielsweise bei der zuvor beschriebenen ophthalmologischen Anwendung erforderlich ist.

WO 2009/050511 beschreibt einen Behälter mit einer Kammer, mit einem Hornhauttransplantat in vorbestimmt verformter Form.

WO 2005/5037136 beschreibt ein Vakuumpaketensystem zum Hydratisieren und/oder Rehydrieren von orthopädischen Transplantatmaterialien wie Allotransplantatmaterialien, Xenotransplantatmaterialien und synthetischen Materialien.

Von ebenfalls besonderer Bedeutung ist für eine erfolgreiche medizinische Anwendung eine sichere Lagerung und ein sicherer Transport des Transplantats oder Implantats. Doch nicht nur eine sichere Lagerung und ein sicherer Transport des Transplantats oder Implantats ist bedeutsam, sondern auch dessen einfache und sichere Bereitstellung aus der Lager- und/oder Transportsituation in die Einsatzsituation. Beschädigungen und/oder Verunreinigungen des Transplantats oder Implantats sind hierbei auszuschließen.

Der vorliegenden Erfindung liegt daher die Aufgabe zugrunde, eine Vorrichtung, eine Aufnahmeeinrichtung und ein Set mit einer solchen Vorrichtung anzugeben, wonach eine sichere Lagerung und/oder ein sicherer Transport sowie eine sichere und einfache Bereitstellung des Transplantats oder Implantats für ein Einbringen in den menschlichen oder in einen tierischen Körper ermöglicht sind.

Erfindungsgemäß ist die voranstehende Aufgabe durch eine Vorrichtung mit den Merkmalen des Anspruchs 1, durch eine Aufnahmeeinrichtung mit den Merkmalen des Anspruchs 8 und durch ein Set mit den Merkmalen des Anspruchs 11 gelöst.

In erfindungsgemäßer Weise ist zunächst erkannt worden, dass eine geeignet ausgestaltete Vorrichtung sowohl zur Lagerung als auch zum Transport eines Transplantats oder Implantats in hervorragender Weise geeignet sein kann. Hierzu weist die Vorrichtung ein mit einem Medium, vorzugsweise Nährmedium, befüllbaren Behälter und eine in dem Behälter anordenbare Aufnahmeeinrichtung für das Transplantat oder Implantat auf. Das Transplantat oder Implantat ist also in einer separaten Aufnahmeeinrichtung in dem Behälter angeordnet, wobei die Aufnahmeeinrichtung einen Aufnahmeraum für das Transplantat oder Implantat und zwei zu dem Aufnahmeraum führende Durchgänge aufweist. Von diesen beiden Durchgängen ist mindestens einer für ein Ein- und/oder Ausbringen des Transplantats oder Implantats in den oder aus dem Aufnahmeraum dimensioniert. Ein Ein- und/oder Ausbringen des Transplantats oder Implantats in den oder aus dem Aufnahmeraum erfolgt hierbei durch denselben Durchgang. Zur sicheren Positionierung des Transplantats oder Implantats im Aufnahmeraum weist die Vorrichtung des Weiteren jeweils eine Verschlusseinrichtung für die beiden Durchgänge auf. Hierdurch ist gewährleistet, dass das Transplantat oder Implantat nicht aus dem Aufnahmeraum in den Behälter bzw. in das in dem Behälter während einer Lagerung und eines Transports üblicherweise angeordnete Medium gelangt. Hierdurch ist eine sichere Handhabung des Transplantats oder Implantats mittels der Aufnahmeeinrichtung und gleichzeitig ein geschütztes Unterbringen des Transplantats oder Implantats mit der Aufnahmeeinrichtung in dem Behälter gewährleistet. In weiter erfindungsgemäßer Weise ist mindestens eine der beiden Verschlusseinrichtungen für das Medium - nicht jedoch für das Transplantat oder Implantat - durchlässig. Dies ermöglicht einen sicheren Kontakt des Transplantats oder Implantats mit dem Medium während der Lagerung und/oder des Transports, da stets eine ausreichende Menge an Medium durch die für das Medium durchlässige Verschlusseinrichtung in den Aufnahmeraum zu dem Transplantat oder Implantat gelangen kann. Dabei kann im Rahmen einer besonderen Ausführungsform eine der beiden Verschlusseinrichtungen durch eine Verengung der Aufnahmeeinrichtung oder eines Durchgangs gebildet sein. Insoweit kann eine der beiden Verschlusseinrichtungen als integraler Bestandteil der Aufnahmeeinrichtung realisiert sein.

Die erfindungsgemäße Vorrichtung ist aufgrund der separaten Handhabbarkeit der Aufnahmeeinrichtung mit dem darin angeordneten Transplantat oder Implantat auch in hervorragender Weise geeignet, das Transplantat oder Implantat nach einer Lagerung und/oder einem Transport für den Operationseinsatz und damit für ein Einbringen in den menschlichen oder tierischen Körper bereitzustellen.

Folglich ist mit der erfindungsgemäßen Vorrichtung eine Vorrichtung angegeben, mit der eine sichere Lagerung und/oder ein sicherer Transport sowie eine sichere und einfache Bereitstellung des Transplantats oder Implantats für ein Einbringen in den menschlichen oder tierischen Körper ermöglicht sind.

Im Hinblick auf eine besonders sichere und einfache Bereitstellung des Transplantats oder Implantats und im Hinblick auf eine besonders einfache konstruktive Ausgestaltung der Aufnahmeeinrichtung kann die Aufnahmeeinrichtung als Hülse oder Zylinder ausgebildet sein. Dabei ist der Aufnahmeraum für das Transplantat oder Implantat durch den Innenraum der Hülse oder des Zylinders und sind die beiden Durchgänge durch die endseitigen Öffnungen der Hülse oder des Zylinders gebildet. Dabei sind diese Durchgänge üblicherweise gleich groß dimensioniert, so dass ein Ein- und/oder Ausbringen des Transplantats oder Implantats in den oder aus dem Aufnahmeraum durch beide Durchgänge erfolgen kann. Insoweit muss nicht auf eine passende Orientierung der Aufnahmeeinrichtung zum Ein- und/oder Ausbringen des Transplantats oder Implantats geachtet werden.

In besonders einfacher und flexibler Weise können die Verschlusseinrichtungen auf die Aufnahmeeinrichtung aufsteckbar sein. Hierdurch ist eine besonders einfache Handhabung der Vorrichtung gewährleistet, wobei das Aufstecken der Verschlusseinrichtungen auf die Aufnahmeeinrichtung üblicherweise per Hand oder mittels einfachster Instrumente erfolgen kann. In vorteilhafter Weise können die Verschlusseinrichtungen als Manschetten ausgebildet sein, die zumindest teilweise auf die Aufnahmeeinrichtung aufgeschoben oder aufgesteckt sein können.

Im Hinblick auf eine sehr einfache Positionierung der Verschlusseinrichtungen auf der Aufnahmeeinrichtung können die Verschlusseinrichtungen aus zumindest geringfügig elastischem Material, vorzugsweise aus Kunststoff, ausgebildet sein. Aufgrund der Elastizität des Materials der Verschlusseinrichtungen können die Verschlusseinrichtungen bei geeigneter Dimensionierung unter Vorspannung auf der Aufnahmeeinrichtung sitzen. Eine Mehrfachverwendung der Verschlusseinrichtungen ist möglich.

Die Verschlusseinrichtungen können grundsätzlich auf unterschiedliche Art und Weise ausgebildet sein, wobei zusätzliche Funktionen der Verschlusseinrichtungen - neben dem Verschließen des jeweiligen Durchgangs - mit der jeweiligen Verschlusseinrichtung realisiert werden können. In besonders vorteilhafter Weise kann mindestens eine der beiden Verschlusseinrichtungen und können vorzugsweise beide Verschlusseinrichtungen ein Gitter zur Bereitstellung der Durchlässigkeit für das Medium aufweisen. Bei im Aufnahmeraum positioniertem Transplantat oder Implantat stellt eine derartige Verschlusseinrichtung einerseits einen sicheren Schutz gegen ein unbeabsichtigtes Herausrutschen des Transplantats oder Implantats aus dem Aufnahmeraum und andererseits die für ein ausreichendes Benetzen oder Spülen des Transplantats oder Implantats mit einem Medium erforderliche Durchlässigkeit für das Medium bereit. Für ein besonders sicheres Einleiten des Mediums in den Aufnahmeraum oder Durchspülen durch den Aufnahmeraum weisen beide Verschlusseinrichtungen ein derartiges Gitter auf. Das Gitter kann in vorteilhafter Weise integraler Bestandteil der jeweiligen Verschlusseinrichtung sein.

Im Hinblick auf weitere Funktionen kann die Aufnahmeeinrichtung oder mindestens eine der beiden Verschlusseinrichtungen zum Anschluss einer Manschette oder eines Adapters oder mit einem integrierten Adapter oder Adapterbereich derart ausgebildet sein, dass ein Anschließen eines Schlauchs, einer Spritze, vorzugsweise einer Einwegspritze, oder einer Einrichtung zum Zu- und/oder Abführen des Mediums und/oder eines weiteren fluiden Mediums, vorzugsweise einer Färbelösung, ermöglicht ist. Zum einen kann also die Aufnahmeeinrichtung zum Anschluss einer Manschette oder eines Adapters oder mit einem integrierten Adapter oder Adapterbereich ausgebildet sein. Zum anderen kann diese Adaptionsmöglichkeit durch eine bereits mit der Aufnahmeeinrichtung gekoppelte Verschlusseinrichtung bereitgestellt sein. Beispielsweise kann der Anschluss eines Schlauchs mit einer daran angeschlossenen Spritze ein Einbringen einer Färbelösung in den Aufnahmeraum ermöglichen. Dabei kann das schon vorher im Aufnahmeraum befindliche Medium herausgespült oder mit der Färbelösung vermischt werden. Bei einer alternativen Anwendung kann mittels des angeschlossenen Schlauchs und der angeschlossenen Spritze durch Erzeugen einer Sogwirkung oder Schubwirkung das Transplantat oder Implantat im Aufnahmeraum bewegt oder aus dem Aufnahmeraum heraus bewegt werden. Hierzu ist dann eine Verschlusseinrichtung mit einem Gitter durch eine Verschlusseinrichtung ohne Gitter zu ersetzen. Bei einer weiteren Anwendung kann eine bekannte Kartusche oder Hülse zum Bereitstellen und zum Einbringen eines Transplantats oder Implantats mit der Aufnahmeeinrichtung gekoppelt werden und an diese Kartusche oder Hülse ein Schlauch mit einer Spritze angeschlossen werden, so dass das Transplantat oder Implantat über eine Sog- oder Schubwirkung bei Betätigen des Kolbens der Spritze in die Kartusche oder Hülse bewegt werden kann. Alle vorgenannten Anwendungen lassen sich auf sehr einfache Weise mittels geeigneten Verschlusseinrichtungen, Manschetten, Adaptern oder Adapterbereichen realisieren, wobei anstelle einer Spritze alternativ grundsätzlich eine geeignete Einrichtung zum Zu- und/oder Abführen des Mediums und/oder eines weiteren fluiden Mediums verwendet werden kann.

Die Aufnahmeeinrichtung kann in vorteilhafter Weise aus Kunststoff, Glas oder Metall ausgebildet sein. Bei der zugrundeliegenden Materialwahl ist auf den jeweiligen Anwendungsfall abzustellen. Beispielsweise ermöglicht eine transparente Ausgestaltung eine sichere Beobachtung und Überwachung der Position des im Aufnahmeraum angeordneten Transplantats oder Implantats. Eine Ausgestaltung aus Glas oder Metall ermöglicht eine sichere Wiederverwendung der Aufnahmeeinrichtung, da Aufnahmeeinrichtungen aus diesen Materialien besonders einfach gereinigt und/oder sterilisiert werden können. Besonders kostengünstig ist üblicherweise eine Ausgestaltung aus Kunststoff, wobei in diesem Fall eine Einmalanwendung in Frage kommt.

Die voranstehende Aufgabe wird des Weiteren durch eine Aufnahmeeinrichtung mit den Merkmalen des nebengeordneten Anspruchs 8 gelöst, wobei die Aufnahmeeinrichtung jeweils eine Verschlusseinrichtung für die beiden Durchgänge aufweist, von denen mindestens eine Verschlusseinrichtung für das Medium durchlässig ist. Hinsichtlich der Vorteile einer derartigen Aufnahmeeinrichtung wird zur Vermeidung von Wiederholungen auf die voranstehenden Erläuterungen verwiesen, denen die Vorteile der Aufnahmeeinrichtung entnehmbar sind.

In besonders vorteilhafter Weise kann an einen der beiden Durchgänge eine Kartusche oder eine Hülse mit zwei gegenüberliegenden, endseitigen Öffnungen angekoppelt sein, wobei die erste Öffnung einen größeren Durchmesser aufweist als die zweite Öffnung. Diese Kombination aus Aufnahmeeinrichtung und angekoppelter Kartusche oder Hülse ermöglicht nicht nur eine sichere Lagerung oder einen sicheren Transport eines Transplantats oder Implantats, sondern auch dessen einfache Bereitstellung für ein Einbringen des Transplantats oder Implantats, beispielsweise mittels einer an den anderen der beiden Durchgänge der Aufnahmeeinrichtung angekoppelten Spritze oder Einrichtung zum Zu- und/oder Abführen des Mediums und/oder eines weiteren fluiden Mediums.

Die voranstehende Kombination aus Aufnahmeeinrichtung und Kartusche oder Hülse kann einem Operateur mit dem darin angeordneten Transplantat oder Implantat zur direkten Einbringung des Transplantats oder Implantats in den menschlichen oder tierischen Körper bereitgestellt werden.

Die Kartusche oder Hülse kann dabei in weiter vorteilhafter Weise derart ausgestaltet sein, dass der äußere Rand der zweiten Öffnung in die Düse einer Spritze einsteckbar ist und/oder dass die erste Öffnung auf die Düse derselben oder einer anderen Spritze aufsteckbar ist. Im Falle eines Einsteckens des äußeren Rands der zweiten Öffnung in die Düse einer Spritze kann über die Spritze ein Unterdruck erzeugt werden, der das Transplantat oder Implantat aus einer an die erste Öffnung angekoppelten Aufnahmeeinrichtung in die Kartusche oder Hülse hinein saugt. Daraufhin kann die Hülse oder Kartusche von der Aufnahmeeinrichtung abgekoppelt werden und mit der ersten Öffnung auf die Düse derselben Spritze oder einer anderen Spritze aufgesteckt werden. Das Transplantat oder Implantat ist damit für ein Einbringen in den menschlichen oder tierischen Körper bereitgestellt, wobei ein Betätigen der Spritze einen geeigneten Schub zum Einbringen des Transplantats oder Implantats in den menschlichen oder tierischen Körper erzeugen kann.

Alternativ hierzu kann eine Spritze an das der Kartusche oder Hülse entgegengesetzte Ende einer mit einer Kartusche oder Hülse gekoppelten Aufnahmeeinrichtung angekoppelt werden. Hierdurch wäre eine Kombination aus miteinander gekoppelter Kartusche oder Hülse, Aufnahmeeinrichtung und Spritze bereitgestellt. Die Kopplung zwischen diesen drei Elementen kann mittels geeigneter Manschetten und/oder Adaptern erfolgen. Ein Einschieben des Kolbens der Spritze übt in diesem Fall eine Schubwirkung auf das Transplantat oder Implantat im Aufnahmeraum der Aufnahmeeinrichtung aus, so dass dieses in die Kartusche oder Hülse bewegt und noch weiter in den menschlichen oder tierischen Körper eingebracht oder eingespritzt werden kann. In diesem Fall ist ein Abkoppeln der Kartusche oder Hülse von der Aufnahmeeinrichtung vor einem Einbringen des Transplantats oder Implantats in den Körper nicht erforderlich.

Die voranstehende Aufgabe wird ebenfalls durch ein Set mit den Merkmalen des Anspruchs 11 gelöst. Ein derartiges Set umfasst eine Vorrichtung nach einem der voranstehenden Ansprüche, wobei sich diese Vorrichtung zur sicheren und komfortablen Bereitstellung in sterilisiertem Zustand oder in einer zur Sterilisierung geeigneten Verpackung befindet. Hinsichtlich der Vorteile der von dem Set umfassten Vorrichtung wird zur Vermeidung von Wiederholungen auf die voranstehenden Erläuterungen der erfindungsgemäßen Vorrichtung verwiesen. Die dort beschriebenen vorteilhaften Wirkungen treffen in gleicher Weise für das erfindungsgemäße Set zu.

In weiter vorteilhafter Weise kann das Set zusätzlich eine Spritze und einen zur Verbindung mit der Vorrichtung geeigneten Adapter und/oder Schlauch aufweisen, so dass unterschiedliche Anwendungen der Bestandteile des Sets ermöglicht sind.

Alternativ oder zusätzlich kann die Aufnahmeeinrichtung bereits ein Transplantat oder Implantat enthalten. Insoweit kann ein gebrauchsfertiges Set im Rahmen der vorliegenden Erfindung bereitgestellt werden.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung bevorzugter Ausführungsbeispiele der erfindungsgemäßen Vorrichtung, der erfindungsgemäßen Aufnahmeeinrichtung und des erfindungsgemäßen Sets zu verweisen. In Verbindung mit der Erläuterung der bevorzugten Ausführungsbeispiele anhand der Zeichnung werden auch im Allgemeinen bevorzugte Ausgestaltungen und Weiterbildungen der Lehre erläutert. In der Zeichnung zeigen
- Fig. 1: in einer schematischen Darstellung ein Ausführungsbeispiel einer erfindungsgemäßen Vorrichtung zur Lagerung und zum Transport eines Transplantats oder Implantats mit einer angeschlossenen Spritze,
- Fig. 2: in einer schematischen Darstellung das Ausführungsbeispiel aus Fig. 1, wobei eine Verschlusseinrichtung gegen eine zur Adaption geeignete Manschette ausgetauscht wird,
- Fig. 3: in einer schematischen Darstellung eine in dem Behälter angeordnete Aufnahmeeinrichtung mit der gemäß Fig. 2 angekoppelten Manschette und einer mittels der Manschette angekoppelten Kartusche oder Hülse zum Bereitstellen und zum Einbringen des Transplantats in den Körper,
- Fig. 4: in einer schematischen Darstellung die Anordnung aus Fig. 3, wobei zusätzlich ein Schlauch und eine Spritze an die Kartusche oder Hülse angekoppelt sind,
- Fig. 5: in einer schematischen Darstellung die Anordnung aus Fig. 4, wobei das Transplantat mittels eines Betätigens der Spritze in die Kartusche oder Hülse bewegt worden ist,
- Fig. 6: in einer schematischen Darstellung die Anordnung aus Fig. 5, wobei die Aufnahmeeinrichtung und die Manschette von der Kartusche abgenommen sind,
- Fig. 7: in einer schematischen Darstellung die Anordnung aus Fig. 6, wobei in die Kartusche eine weitere Spritze eingesteckt ist,
- Fig. 8: in einer schematischen Darstellung die Anordnung aus Fig. 7, wobei der Schlauch von der Kartusche abgenommen ist, so dass ein Einbringen des Transplantats oder Implantats in den Körper mittels der Kartusche ermöglicht ist,
- Fig. 9: in einer schematischen Darstellung den Ausgangspunkt einer weiteren Variante zur Handhabung der erfindungsgemäßen Vorrichtung, wobei diese Darstellung der Darstellung gemäß Fig. 1 entspricht,
- Fig. 10: in einer schematischen Darstellung die Anordnung aus Fig. 9, wobei die Spritze mit Adapter abgenommen und auf der entgegengesetzten Seite eine Verschlusseinrichtung gegen eine zur Adaption geeignete Manschette ausgetauscht ist,
- Fig. 11: in einer schematischen Darstellung die Anordnung aus Fig. 10, wobei eine Kartusche zum Bereitstellen und zum Einbringen des Transplantats in den Körper an die gemäß Fig. 10 angekoppelte Manschette angekoppelt ist, und
- Fig. 12: in einer schematischen Darstellung die Anordnung aus Fig. 11, wobei an das der Kartusche abgewandte Ende der Aufnahmeeinrichtung ein Adapter und eine Spritze angekoppelt sind, so dass das Transplantat oder Implantat mit dieser Anordnung direkt in den Körper eingebracht werden kann.

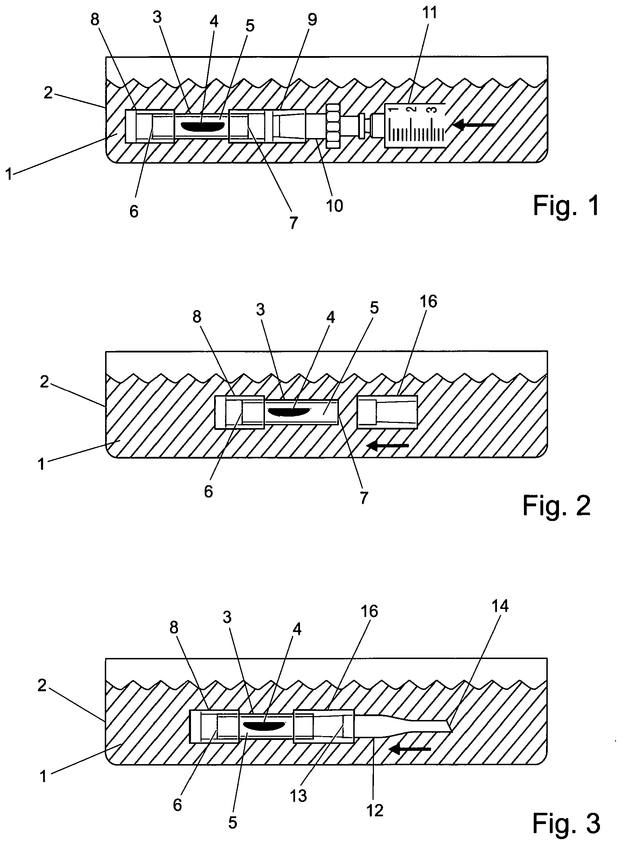

Fig. 1 zeigt in einer schematischen Seitenansicht ein Ausführungsbeispiel einer erfindungsgemäßen Vorrichtung zur Lagerung und zum Transport eines Transplantats oder Implantats. Die Vorrichtung weist einen mit einem Medium 1 befüllbaren Behälter 2 und eine in dem Behälter 2 anordenbare Aufnahmeeinrichtung 3 für das Transplantat 4 oder Implantat auf. Im Folgenden wird der Einfachheit halber nur noch von Transplantat gesprochen, wobei hiermit stets als Alternative ein Implantat gemeint ist.

Die Aufnahmeeinrichtung 3 weist einen Aufnahmeraum 5 für das Transplantat 4 und zwei zu dem Aufnahmeraum 5 führende Durchgänge 6, 7 auf. Die Aufnahmeeinrichtung 3 ist als Zylinder ausgebildet, so dass beide Durchgänge 6, 7 für ein Ein- und/oder Ausbringen des Transplantats 4 in den oder aus dem Aufnahmeraum 5 dimensioniert sind. Beide Durchgänge 6, 7 sind jeweils mit einer Verschlusseinrichtung 8, 9 versehen, wobei beide Verschlusseinrichtungen 8, 9 für das Medium 1 durchlässig sind.

Zum Bestücken der Aufnahmeeinrichtung 3 mit dem Transplantat 4 wird zunächst die zylinderförmige Aufnahmeeinrichtung 3 an einer Seite mit einer Verschlusseinrichtung 9 verschlossen, die für das Medium 1 durchlässig ist, aber ein Durchtreten des Transplantats 4 verhindert. Anschließend wird ein Adapter 10 an die Verschlusseinrichtung 9 angekoppelt und mittels des Adapters 10 eine Spritze 11 angeschlossen, wobei durch ein Herausziehen des Kolbens der Spritze 11 das Transplantat 4 durch den noch unverschlossenen Durchgang 6 eingesaugt wird. Anschließend wird die Aufnahmeeinrichtung 3 mit einer weiteren Verschlusseinrichtung 8 am bis dahin noch offenen Durchgang 6 verschlossen und wird die Spritze 11 mit dem Adapter 10 entfernt. Die Aufnahmeeinrichtung 3 ist damit mit dem Transplantat 4 bestückt und zum Transport bereit.

Bei einer alternativen Methode kann zwischen dem Adapter 10 und der Verschlusseinrichtung 9 ein Schlauch angeschlossen werden. Damit ist die Spritze 11 nicht direkt über den Adapter 10 und die Verschlusseinrichtung 9 an die Aufnahmeeinrichtung 3 angekoppelt, sondern noch zusätzlich über den Schlauch.

Der Behälter 2 kann in Form einer Flasche mit einem Flaschenverschluss, beispielsweise Schraubverschluss, ausgebildet sein. Ein derartiger als Flasche ausgebildeter Behälter 2 kann in vorteilhafter Weise zum Transport oder Versenden des Transplantats 4 verwendet werden. Alternativ hierzu kann der Behälter 2 auch als offene und gegebenenfalls verschließbare Schale ausgebildet sein. Zur einfachen Handhabung der in dem Behälter 2 angeordneten Aufnahmeeinrichtung 3 kann an der Aufnahmeeinrichtung 3 oder an einer Verschlusseinrichtung 8 oder 9 ein Handhabungselement, beispielsweise ein Kunststoff-Streifen, befestigt sein, mit dem die Aufnahmeeinrichtung 3 in einfacher Weise aus der Öffnung des als Flasche ausgebildeten Behälters 2 hinausgezogen werden kann. Das Handhabungselement kann im Bereich des Flaschenhalses oder der Öffnung der Flasche fixiert sein, damit nach einem Abnehmen des Flaschenverschlusses das Handhabungselement auf einfache Weise greifbar und die Aufnahmeeinrichtung 3 aus der Flasche bzw. dem Behälter 2 herausgezogen werden kann. Zur einfachen Handhabung der Aufnahmeeinrichtung 3 kann diese Aufnahmeeinrichtung 3 nach einer Entnahme aus dem Behälter 2 in eine Schale eingelegt werden und kann das in dem Behälter 2 angeordnete Medium 1 ebenfalls in diese Schale gekippt werden. Hierdurch ist eine gute Zugänglichkeit zu der Aufnahmeeinrichtung 3 und zu dem Transplantat 4 gewährleistet.

Die Verschlusseinrichtungen 8 und 9 sind auf die Aufnahmeeinrichtung 3 aufsteckbar und im Wesentlichen als Manschetten ausgebildet. Hierbei sind die Verschlusseinrichtungen 8 und 9 aus einem elastischen Kunststoff hergestellt. Beide Verschlusseinrichtungen 8 und 9 weisen ein Gitter zur Bereitstellung der Durchlässigkeit für das Medium 1 oder ein weiteres Medium auf. Dieses Gitter verhindert jedoch das Austreten des Transplantats 4 aus der Aufnahmeeinrichtung 3.

Fig. 1 zeigt jedoch nicht den oben beschriebenen Beladevorgang der Aufnahmeeinrichtung 3, sondern ein Färben des Transplantats 4. Für das Beladen und das Färben des Transplantats 4 werden üblicherweise verschieden Spritzen 11 verwendet. Sowohl das Beladen der Aufnahmeeinrichtung 3 als auch das Einfärben des Transplantats 4 erfolgen in dem mit dem Medium 1 gefüllten Behälter 2. Der Pfeil in Fig. 1 deutet an, dass eine Färbelösung aus der Spritze 11 durch den Adapter 10 und durch die mit einem Gitter versehene Verschlusseinrichtung 9 hindurch durch den Durchgang 7 in den Aufnahmeraum 5 eingebracht wird. Da beide Verschlusseinrichtungen 8 und 9 für das Medium 1 und auch für die Färbelösung durchlässig sind, aber aufgrund der Ausgestaltung mit einem Gitter die Anordnung des Transplantats 4 im Aufnahmeraum 5 sicherstellen, ist ein Färben und Spülen des Transplantats 4 auf einfache und sichere Weise durch ein Betätigen des Kolbens der Spritze 11 möglich. Während des Spül- und/oder Färbevorgangs verbleibt die gesamte Aufnahmeeinrichtung 3 mit dem Transplantat 4 in dem Medium 1.

Die mit Gittern versehenen Verschlusseinrichtungen 8 und 9 gewährleisten eine hinreichende Zirkulation des Mediums 1 durch die Aufnahmeeinrichtung 3 hindurch während eines Transports der Vorrichtung.

Nach dem Färbevorgang erfolgt gemäß Fig. 2 ein Wechsel der Verschlusseinrichtung 9 gegen eine Manschette 16, die kein Gitter aufweist und ein Hindurchtreten des Transplantats 4 durch den Durchgang 7 und die Manschette 16 ermöglicht. Hierzu wird die Manschette 16 auf die Aufnahmeeinrichtung 3 anstelle der Verschlusseinrichtung 9 aufgesteckt.

In einem nächsten Schritt wird gemäß Fig. 3 eine aus der DE 10 2010 051 458 A1 bekannte Hülse oder Kartusche 12 in einen Adapterbereich der Manschette 16 eingesteckt, um eine Strömungsverbindung vom Aufnahmeraum 5 durch die Manschette 16 hindurch in die Kartusche 12 bereitzustellen. Die Kartusche 12 hat eine größere Öffnung 13, die in der Manschette 16 angeordnet ist. Am entgegengesetzten Ende weist die Kartusche 12 eine Öffnung 14 mit kleinerem Durchmesser als die Öffnung 13 auf. Dieses Ende der Kartusche 12 mit der Öffnung 14 dient dem Einstecken in eine Inzision im menschlichen oder tierischen Körper für ein Einbringen des Transplantats 4.

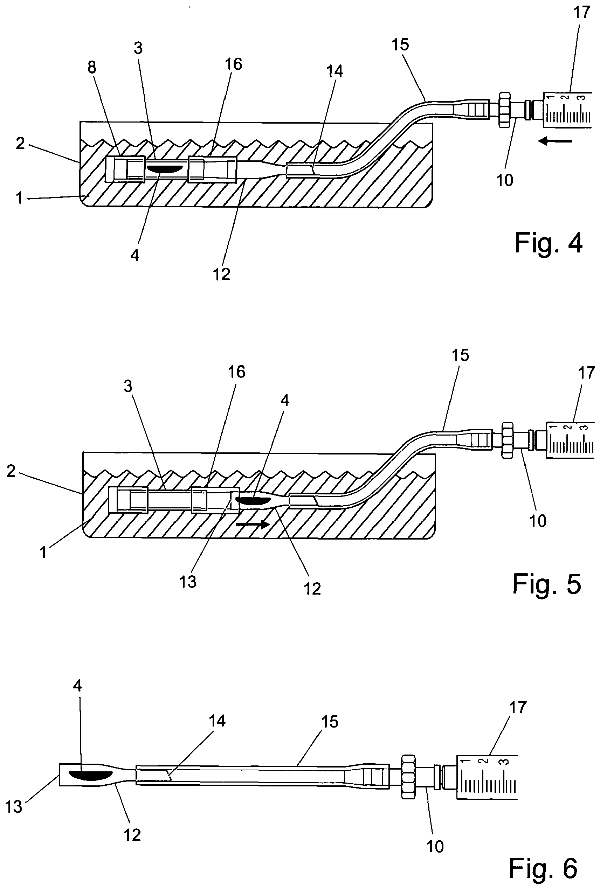

Gemäß Fig. 4 ist in einem nächsten Schritt ein Schlauch 15 über die Öffnung 14 der Kartusche 12 gesteckt. Am anderen Ende des Schlauchs 15 ist ein Adapter 10 mit einer Spritze 17 angeordnet. Die Spritze 17 ist mit etwas Flüssigkeit, beispielsweise einer Kochsalzlösung, gefüllt. Dabei ist auf eine luftblasenfreie Konnektierung zu achten.

Gemäß Fig. 5 ist das Transplantat 4 durch vorsichtiges Zurückziehen des Kolbens der Spritze 17 in die Kartusche 12 bewegt worden. Dabei muss darauf geachtet werden, dass das Transplantat 4 nicht zu schnell eingesogen wird, da es sich sonst im schmalen Teil der Kartusche 12 verklemmen kann, wobei die Gefahr einer Beschädigung des Transplantats 4 besteht.

Sämtliche vorgenannten Schritte finden mit im Medium 1 eingebrachter Aufnahmeeinrichtung 3 statt.

In einem nächsten Schritt können nun der Schlauch 15 und die Manschette 16 mit der Aufnahmeeinrichtung 3 von der Kartusche 12 abgenommen werden. Anschließend kann die Kartusche 12 mit ihrer größeren Öffnung 13 auf eine Düse einer Spritze aufgesteckt werden. Hierdurch ist die Kartusche mit dem darin angeordneten Transplantat 4 für ein Einbringen des Transplantats 4 in den Körper bereit.

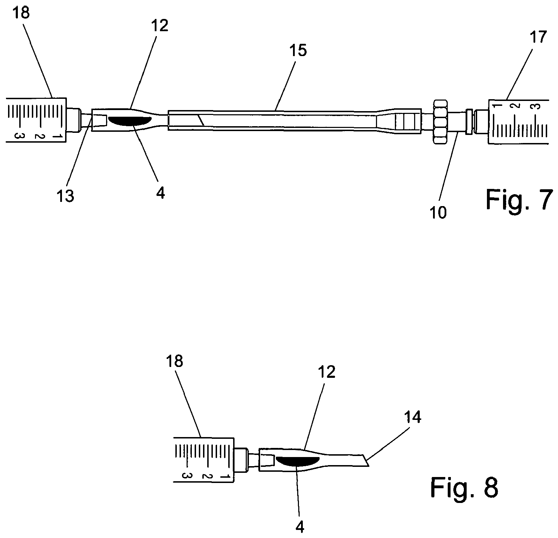

Die Fig. 6 bis 8 zeigen in einer schematischen Darstellung eine Variante einer möglichen Handhabung, die ausgehend von der in Fig. 5 gezeigten Situation mit einer auf eine Spritze 18 aufgesteckten Kartusche 12 endet. Im Konkreten wird gemäß Fig. 6 zunächst die Manschette 16 mit der Aufnahmeeinrichtung 3 von der Kartusche 12 abgenommen. Anschließend wird gemäß Fig. 7 eine weitere Spritze 18 in die größere Öffnung 13 der Kartusche 12 eingesteckt. Anschließend wird gemäß Fig. 8 der Schlauch 15 mit dem Adapter 10 und der Spritze 17 von der Kartusche 12 abgenommen. Die verbleibende Anordnung ist nun bereit für ein direktes Einsetzen des Transplantats 4 in den Körper.

Alternativ zu dem gemäß den Fig. 4 und 5 gezeigten Vorgang des Einbringens des Transplantats 4 in die Kartusche 12 kann beispielsweise eine Spritze - mit oder ohne Schlauch - an den Durchgang 6 der Aufnahmeeinrichtung 3 gemäß der in Fig. 3 gezeigten Situation und Anordnung angekoppelt werden. Für diese Ankopplung der Spritze ist vorzugsweise die Verschlusseinrichtung 8 gegen eine geeignete, für ein Medium 1 oder eine andere Flüssigkeit durchlässige Manschette oder Adaptionseinrichtung zu ersetzen. Bei dieser alternativen Ausführungsform kann das Transplantat 4 durch ein geeignetes Hineinschieben des Kolbens der im Bereich des Durchgangs 6 angekoppelten Spritze durch die Manschette 16 und die Öffnung 13 der Kartusche 12 in die Kartusche 12 bewegt werden. Damit ist das Transplantat 4 ebenfalls für ein Einbringen in den Körper mittels der Kartusche 12 und deren kleineren zweiten Öffnung 14 bereitgestellt. Bei dieser zweiten Bereitstellungsmöglichkeit kann auf ein - im zuvor genannten Fall in den Fig. 4 und 5 beschriebenes - separates Einbringen oder Laden des Transplantats 4 in die Kartusche 12 verzichtet werden. Lediglich durch Anschluss einer geeigneten Spritze am der Kartusche 12 entgegengesetzten Ende der Aufnahmeeinrichtung 3 ist im Wesentlichen schon die Bereitschaft für das Einbringen des Transplantats 4 in den Körper mittels der Kartusche 12 hergestellt.

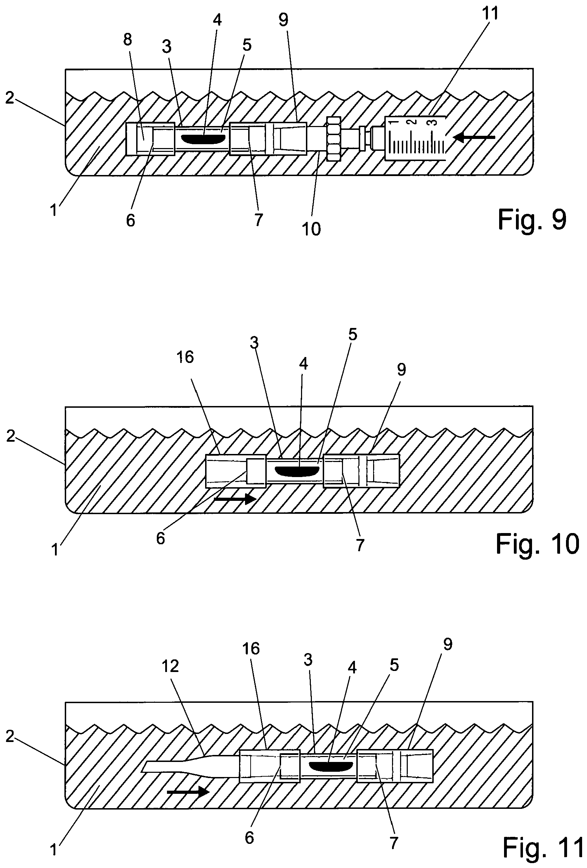

Zu der im vorangehenden Absatz beschriebenen End-Anordnung kann gemäß einem weiteren Ausführungsbeispiel auch die in den Fig. 9 bis 12 gezeigte Abfolge von Anordnungsschritten führen. Dabei wird von der in Fig. 9 dargestellten Anordnung ausgegangen, die der in Fig. 1 dargestellten Anordnung entspricht.

Gemäß Fig. 10 wird die Verschlusseinrichtung 8 durch eine Manschette 16 ersetzt und werden der Adapter 10 und die Spritze 11 von der Verschlusseinrichtung 9 abgenommen.

Gemäß Fig. 11 wird dann eine bereits weiter oben im Detail beschriebene Kartusche 12 an die Manschette 16 angekoppelt.

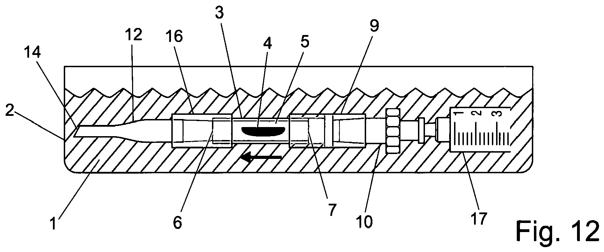

Schließlich wird gemäß Fig. 12 ein Adapter 10 mit einer weiteren Spritze 17 an die Verschlusseinrichtung 9 angekoppelt. Dies ermöglicht das Bewegen des Transplantats 4 durch ein Hineinschieben des Kolbens der Spritze 17 in die Kartusche 12 und danach ein Einbringen des Transplantats 4 in den Körper.

Die erfindungsgemäße Vorrichtung zur Lagerung und zum Transport eines Transplantats 4 ermöglicht ein Beladen der Aufnahmeeinrichtung 3 mit dem Transplantat 4 und eine sichere Lagerung und/oder einen sicheren Transport in dem Behälter 2.

Zum Beladen der Aufnahmeeinrichtung 3 werden beispielsweise die folgenden Teile benötigt: Die Aufnahmeeinrichtung 3, eine Verschlusseinrichtung 9 mit Gitterverschluss und Adaptionsbereich für das Anschließen eines Schlauchs 15 und/oder einer Spritze 11, diesen Schlauch 15, wobei der Schlauch 15 an seinen beiden Enden jeweils einen Adapter 10 aufweist, und eine Spritze 11. Die Verschlusseinrichtung 9 kann in Form einer Manschette mit Gitter und Spülansatz ausgebildet sein. In den Spülansatz kann ein Adapter 10 für den Schlauch 15 oder die Spritze 11 eingesteckt werden.

Zum Einbringen des Transplantats 4 in den Körper können grundsätzlich die beiden oben beschriebenen Varianten angewendet werden.

Dabei werden für die Variante 1 die folgenden Teile benötigt: Verschlusseinrichtung 8 - Manschette mit Gitterverschluss -, Aufnahmeeinrichtung 3, Manschette 16 ohne Gitterverschluss, Kartusche 12, Schlauch 15 mit Adapter 10 und Spritze 17.

Für die Variante 2 werden die folgenden Teile benötigt: Kartusche 12, Manschette 16 ohne Gitterverschluss, Aufnahmeeinrichtung 3, Verschlusseinrichtung 9 mit Gitterverschluss und Adaptionsbereich zum Einstecken eines Adapters, diesen Adapter und Spritze.

Bei vorteilhaften Ausgestaltungen der erfindungsgemäßen Vorrichtung können diese Ausgestaltungen Komponenten gemäß den wie folgt dargestellten Varianten aufweisen:

### Variante 1:

- 8 Manschette mit Gitterverschluss
- 3 Aufnahmeeinrichtung
- 9 Manschette mit Gitterverschluss für Adapter 10 und Spritze 11
- 16 Manschette ohne Gitterverschluss ersetzt die Manschette 9
- 12 Kartusche
- 15 Schlauch
- 10 Adapter
- 11 Spritze
- 17 Spritze

### Variante 2:

- 8 Manschette mit Gitterverschluss
- 3 Aufnahmeeinrichtung
- 9 Manschette mit Gitterverschluss für Adapter 10 und Spritze 11
- 16 Manschette ohne Gitterverschluss ersetzt die Manschette 8
- 12 Kartusche
- 10 Adapter
- 17 Spritze

Im Hinblick auf die Handhabung der in dem Behälter 2 und damit in dem Medium 1 angeordneten Aufnahmeeinrichtung 3, die mit einem Transplantat 4 bestückt ist, kann beispielsweise ein Operateur völlig flexibel handeln. Er kann beispielsweise die zuvor bei verschiedenen Ausführungsbeispielen erläuterten Kopplungs- und Abkopplungsschritte der beschriebenen Komponenten bei im Behälter 2 und damit im Medium 1 angeordneter Aufnahmeeinrichtung 3 vornehmen. Alternativ hierzu kann er-je nach Erfordernis - die Aufnahmeeinrichtung 3 mit dem darin angeordneten Transplantat 4 auch aus dem Medium 1 entnehmen und gegebenenfalls nach Kopplungs- und/oder Abkopplungsvorgängen wieder in das Medium 1 einbringen. Einem Operateur ist hier jegliche Freiheit der Handhabung - je nach Erfordernis und/oder Vorliebe - gegeben.

Hinsichtlich weiterer vorteilhafter Ausgestaltungen der erfindungsgemäßen Vorrichtung, der erfindungsgemäßen Aufnahmeeinrichtung und des erfindungsgemäßen Sets wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele lediglich zur Erörterung der beanspruchten Lehre dienen, diese jedoch nicht auf die Ausführungsbeispiele einschränken.

### Bezugszeichenliste

- 1: Medium
- 2: Behälter
- 3: Aufnahmeeinrichtung
- 4: Transplantat
- 5: Aufnahmeraum
- 6: Durchgang
- 7: Durchgang
- 8: Verschlusseinrichtung
- 9: Verschlusseinrichtung
- 10: Adapter
- 11: Spritze
- 12: Kartusche
- 13: Öffnung
- 14: Öffnung
- 15: Schlauch
- 16: Manschette
- 17: Spritze
- 18: Spritze

## Patentansprüche

1. Vorrichtung zur Lagerung und zum Transport eines Transplantats (4) oder Implantats, mit einem mit einem Medium (1), vorzugsweise Nährmedium, befüllten Behälter (2), mit einer in dem Medium in dem Behälter (2) angeordneten Aufnahmeeinrichtung (3) für das Transplantat (4) oder Implantat, wobei die Aufnahmeeinrichtung (3) einen Aufnahmeraum (5) für das Transplantat (4) oder Implantat und zwei zu dem Aufnahmeraum (5) führende Durchgänge (6, 7) aufweist, von denen mindestens ein Durchgang (6, 7) für ein Ein- und/oder Ausbringen des Transplantats (4) oder Implantats in den oder aus dem Aufnahmeraum (5) dimensioniert ist, und mit jeweils einer Verschlusseinrichtung (8, 9) für die beiden Durchgänge (6, 7), von denen mindestens eine Verschlusseinrichtung (8, 9) für das Medium (1) durchlässig ist.

2. Vorrichtung nach Anspruch 1, **dadurch gekennzeichnet, dass** die Aufnahmeeinrichtung (3) als Hülse oder Zylinder ausgebildet ist.

3. Vorrichtung nach Anspruch 1 oder 2, **dadurch gekennzeichnet, dass** die Verschlusseinrichtungen (8, 9) auf die Aufnahmeeinrichtung (3) aufsteckbar und vorzugsweise als Manschetten ausgebildet sind.

4. Vorrichtung nach einem der Ansprüche 1 bis 3, **dadurch gekennzeichnet, dass** die Verschlusseinrichtungen (8, 9) aus zumindest geringfügig elastischem Material, vorzugsweise aus Kunststoff, ausgebildet sind.

5. Vorrichtung nach einem der Ansprüche 1 bis 4, **dadurch gekennzeichnet, dass** mindestens eine der beiden Verschlusseinrichtungen (8, 9) und vorzugsweise beide Verschlusseinrichtungen (8, 9) ein Gitter zur Bereitstellung der Durchlässigkeit für das Medium (1) aufweist oder aufweisen.

6. Vorrichtung nach einem der Ansprüche 1 bis 5, **dadurch gekennzeichnet, dass** die Aufnahmeeinrichtung (3) oder mindestens eine der beiden Verschlusseinrichtungen (8, 9) zum Anschluss einer Manschette oder eines Adapters (10) oder mit einem integrierten Adapter oder Adapterbereich derart ausgebildet ist, dass ein Anschließen eines Schlauchs, einer Spritze (11), vorzugsweise einer Einwegspritze, oder einer Einrichtung zum Zu- und/oder Abführen des Mediums (1) und/oder eines weiteren fluiden Mediums, vorzugsweise einer Färbelösung, ermöglicht ist.

7. Vorrichtung nach einem der Ansprüche 1 bis 6, **dadurch gekennzeichnet, dass** die Aufnahmeeinrichtung (3) aus vorzugsweise transparentem Kunststoff, Glas oder Metall ausgebildet ist.

8. Set, umfassend eine Vorrichtung nach einem der Ansprüche 1 bis 7 in sterilisiertem Zustand oder in einer zur Sterilisierung geeigneten Verpackung.

9. Set nach Anspruch 8, **dadurch gekennzeichnet, dass** das Set zusätzlich eine Spritze (11) und einen zur Verbindung mit der Vorrichtung geeigneten Adapter (10) und/oder Schlauch aufweist und/oder dass die Aufnahmeeinrichtung (3) bereits ein Transplantat (4) oder Implantat enthält.

## Claims

1. Device for storing and transporting a graft (4) or an implant, comprising a container (2) that is filled with a medium (1), preferably a culture medium, and a receptacle (3), which is arranged in the medium inside the container (2), for the graft (4) or implant, wherein the receptacle (3) comprises a receiving space (5) for the graft (4) or implant and two passages (6, 7) that lead to the receiving space (5), of which at least one passage (6, 7) is dimensioned to introduce and/or remove the graft (4) or implant into or out of the receiving space (5), and said device comprising one sealing means (8, 9) for each of the two passages (6, 7), at least one of which sealing means (8, 9) is permeable to the medium (1).

2. Device according to claim 1, **characterised in that** the receptacle (3) is formed as a sheath or cylinder.

3. Device according to either claim 1 or claim 2, **characterised in that** the sealing means (8, 9) can be slid onto the receptacle (3) and are preferably formed as sleeves.

4. Device according to any of claims 1 to 3, **characterised in that** the sealing means (8, 9) are made of at least slightly resilient material, preferably plastics material.

5. Device according to any of claims 1 to 4, **characterised in that** at least one of the two sealing means (8, 9), and preferably both sealing means (8, 9), comprises or comprise a mesh for providing the permeability to the medium (1).

6. Device according to any of claims 1 to 5, **characterised in that** the receptacle (3) or at least one of the two sealing means (8, 9) is formed to be connected to a sleeve or an adaptor (10) or to have an integrated adapter or adapter region, such that it is made possible for a tube, a syringe (11), preferably a disposable syringe, or a device for supplying and/or discharging the medium (1) and/or an additional fluid medium, preferably a staining fluid, to be connected.

7. Device according to any of claims 1 to 6, **characterised in that** the receptacle (3) is made of preferably transparent plastics material, glass, or metal.

8. Set, comprising a device according to any of claims 1 to 7 in a sterilised condition or in packaging that is suitable for sterilisation.

9. Set according to claim 8, **characterised in that** the set additionally comprises a syringe (11) and an adapter (10) and/or tube that is suitable for connecting to the device, and/or **in that** the receptacle (3) already contains a graft (4) or an implant.

## Revendications

1. Dispositif de stockage et de transport d'un transplant (4) ou d'un implant, avec un récipient (2) rempli d'un milieu (1), de préférence un milieu nutritif, avec un système de réception (3), destiné au transplant (4) ou à l'implant, disposé dans le milieu dans le récipient (2), le système de réception (3) présentant un espace de réception (5) destiné au transplant (4) ou à l'implant et deux passages (6, 7) conduisant à l'espace de réception (5), dont au moins un passage (6, 7) est dimensionné pour l'introduction et/ou l'extraction du transplant (4) ou de l'implant dans ou hors de l'espace de réception (5), et avec respectivement un système de fermeture (8, 9) pour les deux passages (6, 7), dont au moins un système de fermeture (8, 9) est perméable au milieu (1).

2. Dispositif selon la revendication 1, **caractérisé en ce que** le système de réception (3) est constitué en tant que manchon ou cylindre.

3. Dispositif selon la revendication 1 ou 2, **caractérisé en ce que** les systèmes de fermeture (8, 9) peuvent être enfichés sur le système de réception (3) et sont constitués de préférence en tant que manchettes.

4. Dispositif selon l'une des revendications 1 à 3, **caractérisé en ce que** les systèmes de fermeture (8, 9) sont constitués d'un matériau au moins légèrement élastique, de préférence de matière plastique.

5. Dispositif selon l'une des revendications 1 à 4, **caractérisé en ce qu'**au moins un des deux systèmes de fermeture (8, 9) et de préférence les deux systèmes de fermeture (8, 9) présente ou présentent une grille destinée à assurer la perméabilité au milieu (1).

6. Dispositif selon l'une des revendications 1 à 5, **caractérisé en ce que** le système de réception (3) ou au moins un des deux systèmes de fermeture (8, 9) est constitué pour le raccordement d'une manchette ou d'un adaptateur (10) ou avec un adaptateur ou une zone d'adaptateur intégré(e) de telle sorte qu'un raccordement d'un tuyau, d'une seringue (11), de préférence d'une seringue à usage unique, ou d'un système destiné à l'amenée et/ou à l'évacuation du milieu (1) et/ou d'un autre milieu fluide, de préférence d'une solution colorante, est rendu possible.

7. Dispositif selon l'une des revendications 1 à 6, **caractérisé en ce que** le système de réception (3) est constitué de matière plastique, de verre ou de métal de préférence transparent(e).

8. Kit, comprenant un dispositif selon l'une des revendications 1 à 7 à l'état stérilisé ou dans un emballage approprié pour la stérilisation.

9. Kit selon la revendication 8, **caractérisé en ce que** le kit présente en plus une seringue (11) et un adaptateur (10) et/ou tuyau approprié pour le raccordement au dispositif et/ou **en ce que** le système de réception (3) contient déjà un transplant (4) ou un implant.
